# EUROPEAN PATENT APPLICATION

(11) **EP 4 173 489 A1**
(43) Date of publication of application: **03.05.2023**
(21) Application number: 21382983.1
(22) Date of filing: 02.11.2021
(51) Int. Cl.: A23F 3/16, A23C 9/12, A61K 47/69, A23C 21/02, A23L 2/38, A23L 33/105, A61K 8/97, A61K 8/99, A61K 35/741, A61K 36/06, C12F 3/06, A61K 9/127

(54) **BIOCOMPATIBLE EXTRACELLULAR VESICLES OBTAINED FROM FERMENTED FOOD INDUSTRY BY-PRODUCTS, COMPOSITIONS AND APPLICATIONS THEREOF**

(71) Applicant: Fundacio Institut d'Investigació Sanitària Pere Virgili, 43201 Reus (Tarragona) (ES); Centro de Investigación Biomédica en Red, 28029 Madrid (ES); Fundación Imdea Alimentación, 28049 Madrid (ES)
(72) Inventor: GALLART PALAU, Xavier, 43201 REUS (TARRAGONA) (ES); VILELLA CUADRADA, Elisabet, 43201 REUS (TARRAGONA) (ES); SERRA MAQUEDA, Aída, 28049 MADRID (ES); LORCA ROMERO, Cristina, 28049 MADRID (ES)
(74) Representative: Herrero & Asociados, S.L.

(57) **Abstract**

The present invention refers to biocompatible extracellular vesicles, isolated from fermented food industry by-products, wherein the lipidome of the vesicles comprises at least eight families of lipids including fatty acyls (FA), glycerophospholipids (GP), glycerolipids (GL), sphingolipids (SP), saccharolipids (SL), polyketides (PK), sterol lipids (ST) and prenol lipids (PR), with the following ratios between these lipids families: from 0.4 to 1.4 for FA/GL, from 0.2 to 0.4 for FA/GP, from 1.3 to 3.4 for FA/PK, from 4.2 to 7.1 for FA/PR, from 61.2 to 124 for FA/SL, from 0.9 to 3.0 for FA/SP and from 0.9 to 1.6 for FA/ST. The present invention also refers to a method for the isolation of said vesicles, compositions comprising the vesicles and applications thereof.

## Description

### FIELD OF THE INVENTION

The present invention relates to Extracellular Vesicles (EVs) obtained from fermented food industry by-products. The invention also contemplates the method for obtaining said EVs, compositions and applications thereof.

### BACKGROUND OF THE INVENTION

Extracellular vesicles (EVs) are implicated on the mediation of intercellular communication whithin and between organisms. These vesicles are released to the extracellular space and posses the ability to circulate through biological fluids to target other cells, tissues and organs. Although endogenous EVs have been vastly described and identified in different kingdoms of life, the current available sources of vesicles, and the available vesicles that may act as nanocarriers, present substantial challenges in terms of obtention, yield, scalability, safety and biocompatibility.

The capacity of the lipid molecules to form cell membranes by spontaneous assembling was one of the basic and most outstanding findings in biology, however, beyond membranes these molecules are implicated in the formation of EVs. EVs were initially neglected as 'cellular dust' or 'cell debris' [Margolis, L. & Sadovsky Y. The biology of extracellular vesicles: The known unknowns. PLOS Biol. 17, e3000363 (2019*)].* Though, multidisciplinary research on the biogenesis, nature and applications of these vesicles has disclosed the weightiness of EVs to protect specific cellular cargoes from extracellular hazards and provide them with the most appropriate signaling molecules [Raposo, G. & Stoorvogel W. Extracellular vesicles: exosomes, microvesicles, and friends. J. Cell Biol. 200, 373-83 (2013)]. To substantially contribute to intercellular communication, EVs are based on resilient and efficient lipid membranes, include specific molecular cargoes and present preset membrane-embedded signaling molecules [Gallart-Palau, X., Serra, A. & Sze, S.K. Enrichment of extracellular vesicles from tissues of the central nervous system by PROSPR. Mol. Neurodegener. 11, 41 (2016*)].* It is of note that EVs are released and taken up by almost every cell type in the different kingdoms of life [Doyle, L.M. & Wang, M.Z. Overview of Extracellular Vesicles, Their Origin, Composition, Purpose, and Methods for Exosome Isolation and Analysis. Cells 8, 727(2019*)].*

EVs have been proposed as essential functional performers in diverse health and disease conditions, and some of these implications have already been discovered *[*Malloci, M. et al. Extracellular Vesicles: Mechanisms in Human Health and Disease. Antioxid. Redox Signal 30, 813-856 (2019*)].* Similarly, EVs have been identified as an optimal source of organic biocompatible nanocarriers with a growing interest on their potential applications within several areas of the bioeconomy and biomedical fields (in e.g., food and food supplement industries, biotech industries, pharmacy, etc.) *[*Meng, W. et al. Prospects and challenges of extracellular vesicle-based drug delivery system: considering cell source. Drug Deliv. 27, 585-598 (2020*)].* In particular, exosomes, with a global averaged diameter <170-200 nm, have been vastly researched and identified as the best current available source of organic nanocarriers [Wang, Y., Zhang, Y., Cai, G. & Li Q. Exosomes as Actively Targeted Nanocarriers for Cancer Therapy. Int. J. Nanomedicine 15, 4257-4273 (2020*)].*

Although these vesicles display advantageous and interesting features as nanocarriers, including low antigenicity, ability to trespass biological barriers [Kang, M., Jordan, V., Blenkiron, C. & Chamley, LW. Biodistribution of extracellular vesicles following administration into animals: A systematic review. J. Extracell. Vesicles 10, e12085 (2021*)],* stable circulatory capacity and organ tropism, applicability of EVs as optimal nanocarriers still faces substantial challenges [*Meng, W. et al*]. Among these challenges, safety, scalability and identification of biocompatible physicochemical features stand out *[*Gangadaran, P. & Ahn, B. C. Extracellular Vesicle- and Extracellular Vesicle Mimetics-Based Drug Delivery Systems: New Perspectives, Challenges, and Clinical Developments. Pharm. 12, 442 (2020*)].* This is in part due to the fact that the most researched sources of nanocarrier EVs are immortalized cell lines, a limiting source with significant concerns in terms of human safety and resource availability [Adlerz, K., Patel, D., Rowley, J., Ng, K. & Ahsan, T. Strategies for scalable manufacturing and translation of MSC-derived extracellular vesicles. Stem Cell Res. 48, 101978 (2020*)].* Similarly, the optimal progress of the EVs mimetics field, which consists in laboratory-based generation of artificial EVs and liposomes with biocompatible nanocarrier potentialities, faces substantial challenges based on the current available EVs sources *[*Gangadaran, P. et al. In vivo Non-invasive Imaging of Radio-Labeled Exosome-Mimetics Derived from Red Blood Cells in Mice. Front. Pharm. 9, 817-817 (2018*)*].

Foods are known to contain a vast population of EVs, naturally present and ingested by humans daily *[*Pérez-Bermúdez, P., Blesa, J., Soriano, JM. & Marcilla, A. Extracellular vesicles in food: Experimental evidence of their secretion in grape fruits. Eur. J. Pharm. Sci. 98, 40-50 (2017*)].* Presence of EVs in foods has been demonstrated in multiple sources, including milk and dairy products *[*Kang, E.A. et al. Extracellular Vesicles Derived from Kefir Grain Lactobacillus Ameliorate Intestinal Inflammation via Regulation of Proinflammatory Pathway and Tight Junction Integrity. Biomed 8, 522 (2020*)*; Sukreet, Sonal et al "Isolation of extracellular vesicles from byproducts of cheese making by tangential flow filtration yields heterogeneous fractions of nanoparticles". Journal of Dairy Science 104 (2021 July 1*^{st}]*, edible plants, plant-derived products and fermented foods *[Kang, E.A. et al. (2020)].*

Furthermore, EVs isolated from foods seem to be good candidates to optimally perform as nanocarriers in terms of safety, biocompatibility, stability and target specificity. As an example, exosomes obtained from bovine milk are able to enhance oral bioavailability of chemotheraphy and chemopreventive agents, without relevant immunogenic or pro-inflammatory responses in vivo, while also display enhanced tumor targeting capacity compared to free-administered drug molecules [Munagala, R., Aqil, F., Jeyabalan, J. & Gupta, RC. Bovine milk-derived exosomes for drug delivery. Cancer Lett. 371, 48-61 (2016*)].*

However, foods are an expensive and unsustainable source of EVs at industrial level *[*Akuma, P., Okagu, O.D. & Udenigwe, C.C. Naturally Occurring Exosome Vesicles as Potential Delivery Vehicle for Bioactive Compounds. Front. Sustain. Food Syst. 3(23) (2019*)].* As an example, *Sukreet, et al (2021),* disclose the isolation of EVs from the whey fraction cottage cheese (fresh cheese made by coagulating milk with lactic acid) making by tangential flow filtration, obtaining in this case significantly lower yields of enrichment. Thus, finding a standard approach able to globally isolate EVs from different matrices is also crucial.

The reusage of fermentation product from natural food raw materials was previously proposed in WO2019/229271. In that document, extracellular vesicles were isolated from experimentally fermented natural raw materials. Of note, the raw material or the fermentation product must be free of milk, coloring agents, sugars, preservatives, gluten and alcohol. Further, although this document proposes multiple isolation methods, only a non-scalable strategy was tested for the isolation of the EVs displayed in the examples (isolation kit ExoQuick). Moreover, no biocompatibility and physicochemical properties, besides of particle size, were investigated and described for the EVs derived from these fermented raw materials .

Based on the existing needs and problems of the prior art, the present invention provides with biocompatible extracellular vesicles (EVs) obtained from a novel, sustainable and unexplored source in an optimized and highly scalable manner.

The authors of the present invention has defined, for the first time, the use of fermented food industry by-products (FFIBP) as a circular source of safe biocompatible extracellular vesicles, predominantly of exosomes.

Food industries generate high amounts and a huge variety of by-products that have a negative environmental impact and high cost of management [Girotto, F., Alibardi, L. & Cossu, R. Food waste generation and industrial uses: A review. Waste Manag. 45, 32-41 (2015*)].* Surprisingly, the authors of the invention has shown that those FFIBP and residues are an optimal source of EVs with collateral capacity to contribute to the progress of circular economy, a worldwide priority *[*Moraga, G. et al. Circular economy indicators: What do they measure? Resour. Conserv. Recycl. 146, 452-461 (2019*)].* Circular economy promotes reduction, recycling and reusage of industrial waste, improving sustainability.

The authors of the invention have performed in-depth physicochemical characterization of the obtained by-product-derived EVs (BP-EVs) with special emphasis on lipidome and proteome compositions. The obtained EVs extracellular vesicles show high abundancy of membrane lipids with signaling capacity and complex proteomes resembling endogenous human EVs. These EVs do not show relevant citotoxicity and display excellent biocompatible properties such as capacity to efficiently cross biological barriers, oral and intravenous biovailability and specific organ targeting capacity, assesed through in vivo and in vitro approaches.

### BRIEF DESCRIPTION OF THE DRAWINGS

**Figure 1****.** Measurement by nanoparticle tracking analysis of the particle size of BP-EVs.
**Figure 2****.** Lipid composition of BP-EVs expressed as ratio between fatty acyls (FA) versus glycerolipids (GL), glycerophospholipids (GP), polyketides (PK), prenol lipids (PR), saccharolipids (SL), sphingolipids (SP) and sterol lipids (ST).
**Figure 3****.** Relative abundance of the different main lipid families detected in the lipidome of BP-EVs. Nomenclature used: fatty acyls (FA) versus glycerolipids (GL), glycerophospholipids (GP), polyketides (PK), prenol lipids (PR), saccharolipids (SL), sphingolipids (SP) and sterol lipids (ST).
**Figure 4****.** Averaged distribution of lipids according to their degree on unsaturation identified in lipidomes of BP-EVs.
**Figure 5****.** Averaged total proteins and total tryptic peptides identified by LC-MS/MS from the proteomes of BP-EVs.
**Figure 6****.** Molecular and functional protein categorization of membrane proteins identified in proteomes from BP-EVs.
**Figure 7****.** Number of common proteins identified between BP2-EV proteomes and its food-derived EVs counterpart bovine milk.
**Figure 8****.** Biocompatibility analysis of BP-EVs by assessing in vitro cytotoxicity. In vitro cytotoxicity was assessed in Caco-2 cells seeded in 96-well cell plate at a cell density of 15,000 cells/well treated with different concentrations of BP-EVs (Treatment 1 (T1): 10⁸ particles/ml, Treatment 2 (T2): 10⁹ particles/ml and Treatment 3 (T3): 10¹⁰ particles/ml) by cell viability MTT assay. * Indicates significant differences with p<0.05.
**Figure 9****.** Biocompatibility analysis of BP-EVs assessing whole-body biodistribution. Whole body non-invasive monitoring of the biodistribution 24h upon administration of fluorescently labeled BP-EVs compared to the positive control corresponding to animals treated with the fluorescent. Whole-body distribution quantified by measurement of Radiant Efficiency.
**Figure 10****.** Biocompatibility analysis of BP-EVs assessing oral bioavailability. Whole body non-invasive monitoring of the biodistribution 24h upon administration of fluorescently labeled BP-EVs comparing the oral and intravenous (i.v.) administration ways. Whole-body distribution quantified by measurement of Radiant Efficiency.
**Figure 11****.** Ex-vivo tissue fluorescent imaging at 25h post-oral administration of fluorescently labeled BP-EVs. Total tissue accumulation quantified by measurement of Radiant Efficiency. Only tissues that showed significant differences compared to the positive control corresponding to animals treated with the fluorescent dye are displayed.

### DETAILED DESCRIPTION OF THE INVENTION

In a first embodiment, the present invention refers to biocompatible extracellular vesicles (EVs), isolated from fermented food industry by-products (FFIBPs), herein after, BP-EVs or EVs of the invention.

The EVs of the invention show high abundancy of membrane lipids with signaling capacity and complex proteomes mimicking those circulating in human blood plasma. Specifically, the EVs of the invention are characterized by a lipid profile that comprises at least eight families of lipids including fatty acyls (FA), glycerophospholipids (GP), glycerolipids (GL), sphingolipids (SP), saccharolipids (SL), polyketides (PK), sterol lipids (ST) and prenol lipids (PR). The ratios between these lipids families are from 0.4 to 1.4 for FA/GL, from 0.2 to 0.4 for FA/GP, from 1.3 to 3.4 for FA/PK, from 4.2 to 7.1 for FA/PR, from 61.2 to 124 for FA/SL, from 0.9 to 3.0 for FA/SP and from 0.9 to 1.6 for FA/ST.

The specific lipid pattern of the vesicles of the present invention contributes to the excellent improved biocompatibility and bioavailability properties of said vesicles.

In particular, FA and PR are lipid families abundantly present in all BP-EVs of the invention. These types of lipids have been previously described to be associated with EVs of non-tumorogenic origin. Further, lipidomes of BP-EVs are rich in saturated lipids. The richness in saturated lipids detected in the lipidomes of BP-EVs is associated with the predominance in exosomes, fact in line with the small averaged diameter of BP-EVs. Additionally, analysis of the degree of saturations of the lipidomes of BP-EVs showed an enrichment in polyunsaturated fatty acids (PUFA) with low degree of unsaturations. Richness in PUFA conferes the membrane of BP-EVs with a manifold of dynamic cell signaling abilities linked to decreased stiffness and increased elasticity of membrane.

The membrane proteome of the EVs of the invention contains the metalloenzyme enolase and/or one or more of its isoforms (such as enolase I and enolase II), or enolase associated proteins (such as the regulation of enolase protein 1 or enolase domain containing proteins). The diversity of enolases and enolase-related proteins detected in each BP-EVs population depends on the composition (participating microorganisms) of the initial FFIBPs. Enolases have previously been related to quality control of EVs preparations and may act as indicators of EVs bioactive capacity being bioactive protein cargoes. Enolases were the most ubiquitous proteins present in the BP-EVs.

As used herein, the term "fermented food industry by-products" (FFIBPs), refers to by-products, residues or wastes generated during the industrial production processes of fermented foods. FFIBPs are considered secondary products generated during the manufacturing process of fermented foods and are not produced in the food industry to be used or sold as human food.

The FFIBPs used as source of the EVs of the invention are preferably generated during the production processes of fermented dairy products (such as yogurt, kefir, koumiss, acidophilus milk, fermented cheese, sour cream, ymer or filmjölk), fermented plant-based products (such as non-dairy yogurt, tofu and other soy-fermented products, sauerkraut, kimchi, pickled vegetables, sourdought bread or vinegar) and/or fermented beverages (such as kombucha, water kefir, tepache, beers, wine, whey soda, cider cereal fermented drinks derived from the fermentation of grains such as rice, buckweat, barley, among other or sprits, etc).

These fermented food industry by-products can be liquid, such as brewer's spent yeast or yogurt whey, or solid, such as fermented grape pomace or spent symbiotic culture of bacteria and yeast (SCOBY) derived from the production of kombucha.

These FFIBPs naturally contain the microorganisms responsible of the industrial fermentation.

The industrial fermentation process, as defined in the present invention, is carried out by bacteria, preferably lactic acid bacteria, yeast or fungus species.

In a preferred embodiment, the industrial fermentation is predominantly carried out by probiotic microorganisms (e.g. Saccharomyces cerevisiae or lactic acid bacteria), which confer BP-EVs of the invention with capacity of host immune system reinforcement, opposed to EVs produced by commensal or symbiotic bacteria, which have demonstrated to exert pro-inflammatory responses in essential host organic systems *[*Macia, L., Nanan, R., Hosseini-Beheshti, E. & Grau, GE. Host- and Microbiota-Derived Extracellular Vesicles, Immune Function, and Disease Development. Inter. J. Mol. Sci. 21, 107 (2019*)].*

The diameter of the biocompatible extracellular vesicles of the invention ranges from 30 to 950 nm. Preferably, the 50% of the vesicles have a diameter smaller than 200 nm, which indicate large presence of exosomes in the obtained EVs preparations.

The averaged diameter of the EVs of the invention, together with their molecular composition, (e.g. enrichment in PUFA lipids) indicate large yield of exosomes and high abundancy of membrane lipids with signaling capacity in these vesicles.

BP-EVs of the present invention display all those excellent biocompatible properties previously demonstrated for Food EVs, such as capacity to efficiently cross biological barriers, ability to circulate in organic fluids and specific organ targeting capacity. But, further to all the innate greatness of food EVs, the BP-EVS show a higher availability and sustainability and a significantly reduced cost of obtention together with demonstrated safety in terms of citotoxicity compared to further compromised sources of EVs, as immortalized cell lines.

Moreover, the EVs of the invention are particularly advantageous because they show privileged ability to cross the Blood Brain Barrier (BBB), a key feature in biomedical applications that attempt to target the central nervous system.

Specifically, further to the ability of crossing biological barriers, the EVs of the present invention remain in brain tissues beyond the time-point of 24h, fact that has not been yet demonstrated by other EVs populations and that reinforces the notion that BP-EVs are further biocompatible with the central nervous system constituents.

The Extracellular vesicles of the present invention are intravenously and orally bioavailable, being the oral administration the most optimal administration via to achieve broader whole-body biodistribution of these BP-EVs, and lower accumulation in the liver, which is primarily linked to detoxification.

The EVs of the invention preferentially target bone, brain and liver tissues. These organ targetting patterns differentiate the vesicles of this invention from other vesicles of the state of the art which preferentially have been reported to target the lungs.

Exploitation of industry BPs, as a source of EVs, goes paired with the use of an optimized, scalable and efficient EVs isolation strategy based on industrial scalability, methodological simplicity, lower execution costs and standardization. In particular, EVs of the present invention can be isolated by techniques selected from PRotein Organic Solvent PRecipitation (PROSPR), molecular weight cut-off (MWCO), single-step size exclusion chromatography (SEC) or ultracentrifugation, among others.

In a preferred embodiment, the extracellular vesicles of the invention are isolated by particle size filtration. In a more preferred embodiment, the particle size filtration technique is molecular weight cut off (MWCO). MWCO filtration is the most efficient and simple way to obtain BP-EVs with improved yields even in solid BP sources. Furthermore, this isolation strategy can be easily adapted at industrial level through scaled optimization (based on the parameters summarized below) of its available industrial sibling dubbed as 'tangential flow filtration'.

According to the above, in a second embodiment, the present invention refers to a method for the isolation of extracellular vesicles from FFIBPs, comprising the following steps:
a) Centrifugation, at 4.000-12.000 x g, preferably 6000 x g, during 10-40 min, preferably 35 min, at 3-6°C, preferably 4°C, for liquid FFIBPs samples, or homogenization, to disrupt or lyse up the solid tissue, followed by centrifugation, at 4.000-12.000 x g, preferably 6.000 x g, during 10-40 min, preferably 35 min, at 3-6°C, preferably 4°C, for solid FFIBPs samples, of the FFIBPs to obtain pre-processed FFIBPs,
b) Centrifugation of pre-processed FFIBPs obtained in a) in a spin-filter of 200-500 kDa, preferably 300 kDA, of pore size at 5.000-7.000 x g, preferably 6.000, for 15-45 min, preferably 35 min, at 3 - 6°C, preferably 4°C,
c) Addition of PBS into the same spin-filter and centrifugation at 5.000-7.000 x g, preferably 6.000, for 15-45 min, preferably 35 min, at 3-6 °C, preferably 4°C,
d) Repetition of step c), and
e) Collection of the concentrated BP-EVs sample remaining in the upper side of the filter in d).

The homogenization in step a), when the starting FFIBPs sample is solid, is preferably carried out using a manual glass Dounce homogenizer or automatic tissue homogenizer (until solid particles are reduced < 30%).

The EVs of the present invention can be loaded with a wide range of molecules, including, but being not limited to, bioactive molecules, active compounds, excipients, etc. (those molecules include metabolites, peptides, proteins, lipids, RNAs, DNAs, organic and inorganic compounds, etc.) and their membrane can also be edited by the addition or deletion of different molecules (metabolites, peptides, proteins, lipids, RNAs, DNAs, organic and inorganic compounds, etc).

Accordingly, the EVs of the present invention display advantageous and compelling features as nanocarriers, nanovectors and/or nanocapsules for molecules delivery and/or encapsulation including low antigenicity, ability to trespass biological barriers, stable circulatory capacity and organ tropism. The vesicles can also be used by itself or after their molecular modification to enter endogenous routes of EVs in plant, animal and human cells (e.g. modulation of human cell autophagy mechanisms).

Therefore, in a third embodiment, these abilities position these vesicles as an excellent source of nanocarriers, nanovectors and/or nanocapsules for molecules delivery and/or encapsulation to be used in manifold applications within the novel bioeconomy and biomedical fields, e.g., food and/or food supplement supplement industries for animal or human use, supplements or treatment for plants, biotech industries, pharmacy, cosmetic, etc.

In another embodiment, the present invention refers to a composition comprising a population of the biocompatible extracellular vesicles of the invention.

The biocompatible EVs of the invention, or the composition comprising said EVs, can be used in the manufacture of a food and/or food supplement for animal or human use, a supplement for plants or a cosmetic product.

The composition can be also a pharmaceutical composition, for its use as a medicament, comprising a therapeutic amount of said composition and a pharmaceutically acceptable carrier.

Preferably said composition comprises all excipients/adjuvants/carriers standardly used for enteral or parenteral route of administration, as well as any other route of administration (e.g. nasal, ocular, topic, intravenous, subcutaneous, rectal, vaginal, intrathecal, oral, etc.), such as protective agents against enzymatic digestion in physiological or pathological contexts, bio-compatible substances to stabilize EVs, synthetic lipid-based systems to deliver EVs in multivesicular lyposome bodies, spray formulations for oral/respiratory system use or polymer-based degradable/not degradable gels.

The following experimental section is provided purely by way of illustration and is not intended to limit the scope of the invention as defined in the appended claims. In the following experimental section, reference is made to the appended figures.

### EXAMPLES

### MATERIALS AND METHODS

### Food industry by-products

Four different FFIBPs were tested as potential source of EVs: i) brewer's spent yeast derived from the beer fermentation production (BP1); ii) whey from unbranded plain yogurt (BP2); iii) symbiotic culture of bacteria and yeast (SCOBY) derived from the production of tea fermented drink (kombucha) (BP3) and iv) fermented wine pomace from red grapes derived from the wine fermentation production (BP4). Prior to EVs isolation, FFIBPs were pre-processed as follows: Liquid FFIBPs (BP1 and BP2) were centrifuged at 10,000 x g, 30 min at 5°C, and solid FFIBPs (BP3 and BP4) were previously homogenized using a Dounce homogenizer.

### EVs isolation methods by molecular weight cut-off filtration

Isolation of BP-EVs by MWCO was performed from 3 mL of pre-processed BP by using Vivaspin PES 300 kDa MWCO spin-filters. Spin-filters were centrifuged at 6.000 x g for 35 min at 4°C. After initial centrifugation of 3 mL of BPs sample, the sample remaining in the upper side of the MWCO membrane was washed twice with 10 mL of PBS and spin-filter was centrifuged again using the same conditions. Concentrated BP-EVs sample remaining in the upper side of the filter was collected and stored at - 80 °C for further downstream analysis.

### Nanoparticle tracking analysis

BP-EVs were subjected to extensive morphometric characterization by nanoparticle tracking analysis (NTA). NTA was done as previously described *[*Gallart-Palau, X. et al. Brain-derived and circulating vesicle profiles indicate neurovascular unit dysfunction in early Alzheimer's disease. Brain Pathol. 29, 593-605 (2019*)*].

### Characterization of BP-EVs by lipidomics

BP-EVs were subjected to a liquid-liquid extraction with ice-cold methanol and methyl tert-butyl ether. The organic phase containing the lipids was separated by centrifugation at 1.400 g at 10 °C for 10 min *[*Pizarro, C., Arenzana-Rámila, I., Pérez-del-Notario, N., Pérez-Matute, P. & Gonzá/ez-Sáiz, J-M. Plasma Lipidomic Profiling Method Based on Ultrasound Extraction and Liquid Chromatography Mass Spectrometry. Anal. Chem. 85, 12085-12092 (2013*)].* BP-EVs-derived lipid extracts were subjected to liquid chromatography coupled with tandem mass spectrometry (LC-MS/MS) using a UPLC 1290 series coupled to electrospray ionization quadrupole time-of-flight mass spectrometry (ESI-Q-TOF MS/MS) 6545 (Agilent Technologies, Barcelona, Spain) following a previously published method *[*Castro-Perez, J.M. et al. Comprehensive LC-MSE Lipidomic Analysis using a Shotgun Approach and Its Application to Biomarker Detection and Identification in Osteoarthritis Patients. J. Proteome Res. 9, 2377-2389 (2010*)*; Pradas, I. et al. Metformin induces lipid changes on sphingolipid species and oxidized lipids in polycystic ovary syndrome women. Sci. Rep. 9, 16033 (2019*)].* Identities of interest were then confirmed by tandem mass spectrometry (MS/MS).

### Proteomics characterization of EVs

BP-EVs were lysed and denaturalization of proteins followed by tryptic digestion was carried out as previously described *[*Gallart-Palau, X., Serra, A. & Sze, S.K. Enrichment of extracellular vesicles from tissues of the central nervous system by PROSPR. Mol. Neurodegener. 11, 41 (2016*);* Gallart-Palau, X., Guo, X., Serra, A. & Sze SK. Alzheimer's disease progression characterized by alterations in the molecular profiles and biogenesis of brain extracellular vesicles. Alzheimers Res. Ther. 12, 54 (2020*);* Gallart-Palau, X., Serra, A. & Sze, S.K. System-wide molecular dynamics of endothelial dysfunction in Gram-negative sepsis. BMC Biol. 18, 175 (2020*);* Serra, A., Gallart-Palau, X., Wei, J. & Sze, SK. Characterization of Glutamine Deamidation by Long-Length Electrostatic Repulsion-Hydrophilic Interaction Chromatography-Tandem Mass Spectrometry (LERLIC-MS/MS) in Shotgun Proteomics. Anal. Chem. 88, 10573-10582 (2016*)].* BP-EVs digested proteomes were subjected to LC-MS/MS analysis by using a nanoElute liquid chromatograph (Bruker Daltonics, MA, USA) on-line coupled to a state-of-the-art timsTOF Pro mass spectrometer (Bruker Daltonics, MA, USA) using parallel accumulation - serial fragmentation (PASEF) data acquisition *[*Meier, F. et al. Online Parallel Accumulation-Serial Fragmentation (PASEF) with a Novel Trapped Ion Mobility Mass Spectrometer. Mol. Cell Proteom. 17, 2534-2545 (2018*)].*

Identification of BP-EVs proteomes was carried out using in-house databases that were created by combining the existent proteome available in the National Center for Biotechnology Information (NCBI) from all possible organisms present in every FFIBPs sample based on existing literature [Freitas, M. The Microbiota in Gastrointestinal Pathophysiology: The Benefits of Yogurt, Cultures and Fermentation Ch 24 (Academic Press, Boston 209-223, 2017*;* Coton, M. et al. Unraveling microbial ecology of industrial-scale Kombucha fermentations by metabarcoding and culture-based methods. FEMS Microbiol. Ecol. 93 (2017*);* Kántor, A., Mareček, J., Ivanisšová, E., Terentjeva, M. & Kačániová, M. Microorganisms of Grape Berries. Proc. Latv. Acad. Sci. B Nat. Exact Appl. Sci. 71, 502-508 (2017*)].*

### Cell toxicity assessment by in vitro assays

### Cell culture

Caco-2 cells were cultured in Dulbecco's Modified Eagle's Medium complemented with 10% fetal bovine serum, 1% non-essential amino acids, 1% sodium pyruvate and 1% HEPES buffer solution, at 37°C with 5% CO₂.

### Cell viability MTT Assays

Caco-2 cells were cultured at 15.000 cells per well in a P96 well plate. One day after culturing, the medium was removed and BP-EVs were added at three different concentrations: Treatment 1: 10⁸ particles/mL, Treatment 2: 109 particles/mL and Treatment 3: 10¹⁰ particles/milliliter. Cells were subsequently incubated for 24 hours. After the incubation period, MTT assay was performed. Absorbance was measured at 590 nm using a spectrophotometer.

*In vivo monitoring of BP-EVs bioavailability and biodistribution by fluorescent imaging* All in vivo and ex vivo experiments were carried out at the Animal Experimental Service (SEA) of the Institut de Recerca de l'Hospital de la Santa Creu i Sant Pau. All the in vivo procedures were approved by the Hospital de Sant Pau Animal Ethics Committees and performed in strict accordance with the International Guiding Principles for Animal Research. The three Rs principle [Fenwick, N., Griffin, G. & Gauthier, C. The welfare of animals used in science: how the "Three Rs" ethic guides improvements. Can. Vet. J. 50, 523-530 (2009*)]* and our ethical principle to preferentially minimize animal suffering were in all cases observed.

Fluorescent labeled BP-EVs were prepared fresh prior the in vivo study. BP-EVs resuspended in PBS were labeled with 1 µM of Vybrant^{™} DiD Cell-Labeling Solution. Mice were treated with ∼3 x 10⁹ particles suspended in 200 µL of PBS administered by oral gavage or by i.v. route. Two different controls were carried out; a control of the dye where 200 µL of the unbound dye collected during the MWCO filtration of fluorescent-labeled BP-EVs was administered and a negative control where 200 µL of PBS were administered. Oral bioavailability, whole-body biodistribution and organ targeting capacity of fluorescent-labeled BP-EVs were monitored noninvasively by fluorescent imaging in whole live animals and upon sacrifice by fluorescent imaging of ex-vivo tissues. Fluorescent imaging experiments were performed by using the IVIS Spectrum imaging system (PerkinElmer Life Science, Waltham, MA, USA).

### Data analysis and statistics

Data were analyzed considering a significance threshold of p < 0.05 and were reported as mean ± standard error of mean (SEM), unless otherwise specified.

### RESULTS/EXAMPLES

### Example 1

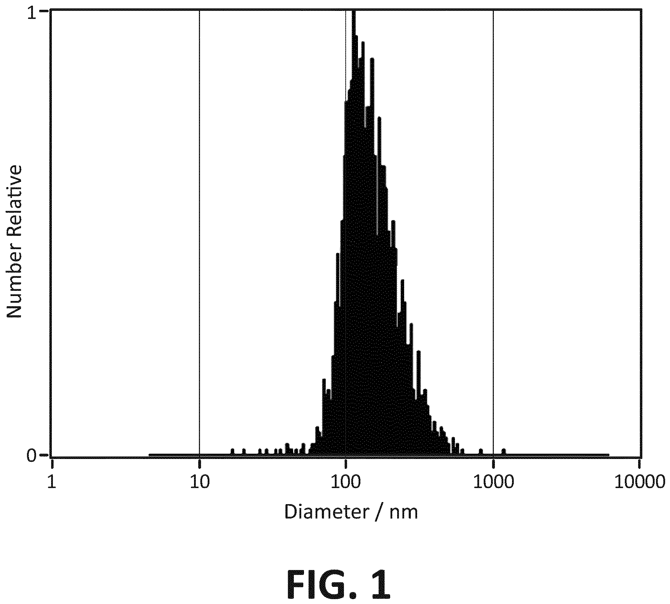

To investigate the viability of the present invention, four different and representative FFIBPs to isolate BP-EVs were used (as described above). Three of these FFIBPs exclusively contained EVs derived from plants and microorganisms (yeast and/or bacterial strains) (BP1, BP3 and BP4), while BP2 was supposed to contain EVs derived from microorganisms and mammalian cells. MWCO, an scalable and simple isolation method, was optimized and tested for the isolation of BP-EVs from the four different FFIBPs. Morphometric characterization of the obtained BP-EVs indicated an averaged diameter ranging from 30 to 950 nm with predominance of particles within the exosomal range, where 50% of the vesicles had a diameter smaller than 200 nm as indicated in **Figure 1**.

### Example 2

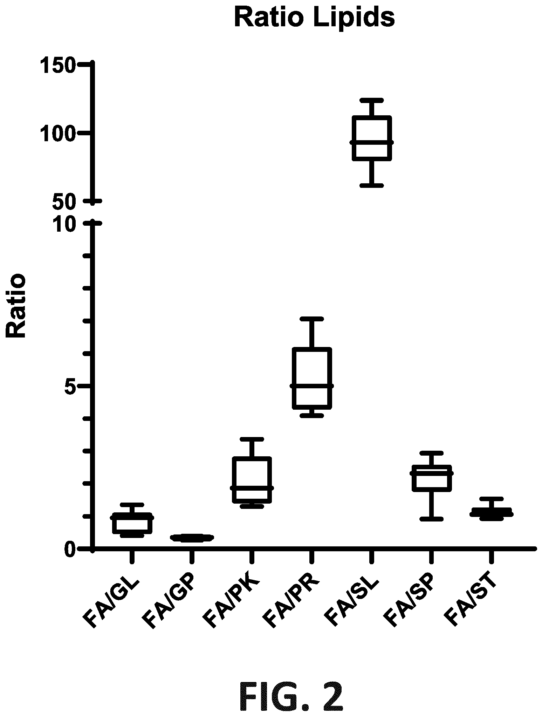

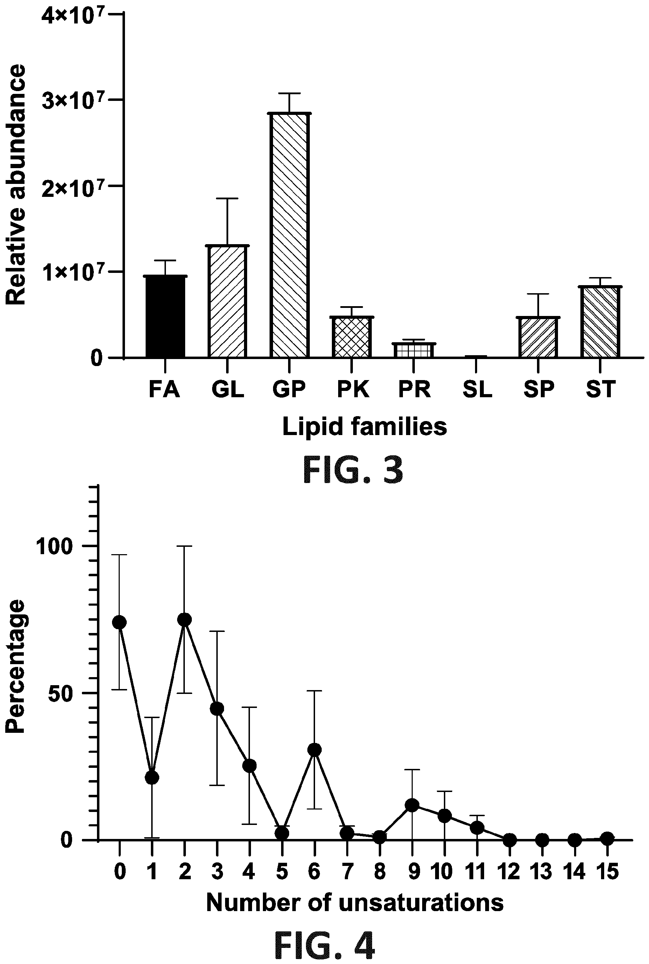

In order to investigate the lipidome composition of BP-EVs, next-generation untargeted lipidomics to define the lipid compositions of the BP-EVs, obtained in example 1, was carried out. Identified molecules were classified in lipid families as shown in **Figure 2** and **3**. Eight lipid families were commonly identified in all BP-EVs investigated, including lipids from the fatty acyls (FA), glycerophospholipids (GP), glycerolipids (GL), sphingolipids (SP), polyketides (PK), sterol lipids (ST), saccharolipids (SL) and prenol lipids (PR) families. FA, GL and GP were the most abundant lipid families (**Figure 3**) being GP the most abundant lipid family in the lipidomes of BP-EVs; lipids from the SL family were consistently identified in all BP-EVs investigated, nonetheless, at very low abundance. The ratios between families of lipids were consistent in all BP-EVs investigated, as detailed in Figure 2 and Table 1.

**Table 1. Detected ranges of lipid ratios.**

| | **BP1** | **BP2** | **BP3** | **BP4** |
|---|---|---|---|---|
| **FA/GL** | 1.1 ± 0.2 | 0.4 ± 0.2 | 1.1 ± 0.4 | 0.7 ± 0.7 |
| **FA/GP** | 0.4 ± 0.0 | 0.3 ± 0.0 | 0.3 ± 0.2 | 0.4 ± 0.1 |
| **FA/PK** | 3.1 ± 0.1 | 1.3 ± 0.3 | 1.7 ± 1.0 | 2.2 ± 1.5 |
| **FA/PR** | 6.1 ± 0.5 | 4.4 ± 0.6 | 4.5 ± 3.0 | 5.7 ± 2.0 |
| **FA/SL** | 113.7 ± 14 | 79.1 ± 31.5 | 92.8 ± 53.2 | 95.1 ± 44.2 |
| **FA/SP** | 2.6 ± 0.3 | 1.7 ± 1.1 | 2.3 ± 1.1 | 2.1 ± 1.4 |
| **FA/ST** | 1.3 ± 0.1 | 1.0 ± 0.3 | 1.1 ± 0.7 | 1.2 ± 0.4 |

Finally, the degree of unsaturation of the lipids from lipidomes of BP-EVs, which identities were successfully validated by LC-MS/MS, was also investigated (**Figure 4**). It was shown that lipidomes of BP-EVs were enriched in saturated fatty acids (SFA), as well as in polyunsaturated fatty acids (PUFA) with low degrees of unsaturation.

### Example 3

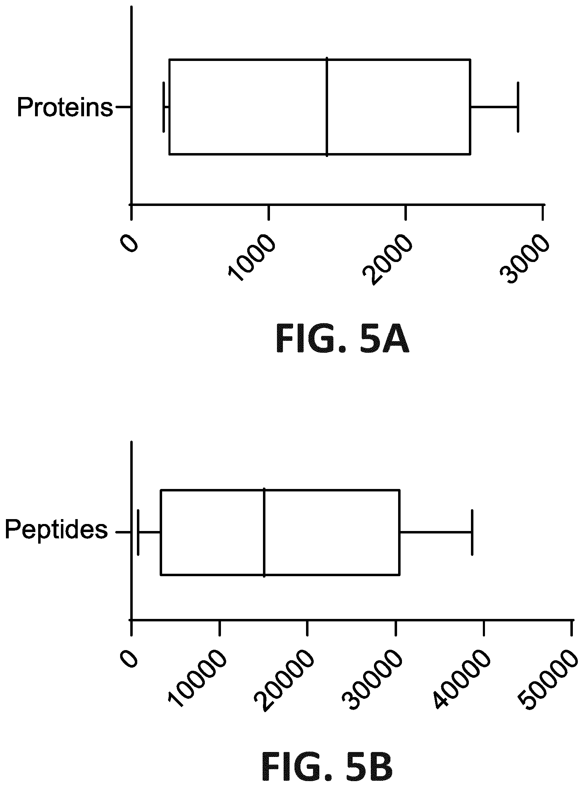

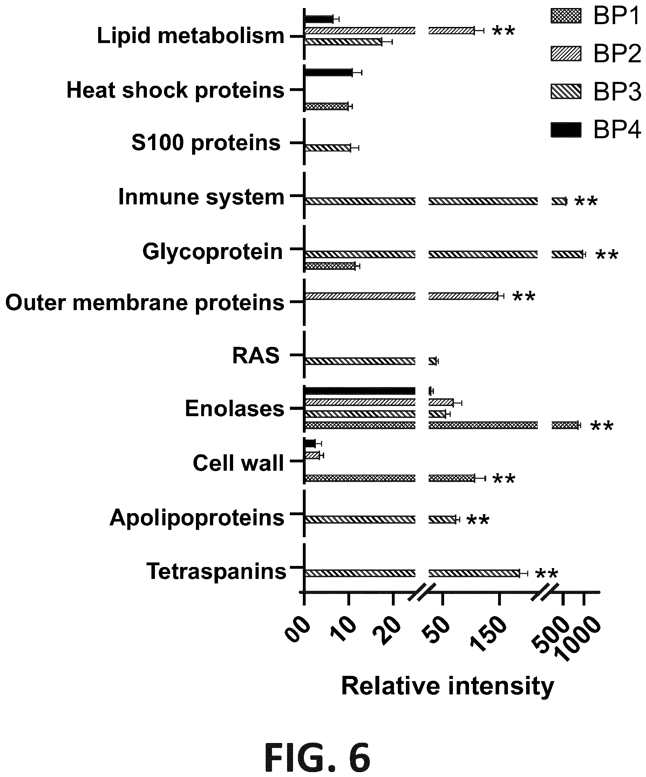

The proteome composition of BP-EVs was also investigated by performing unbiased discovery-driven proteomics by using next-generation four-dimensional state-of-the-art technology *[*Pieters, B. et al. Commercial Cow Milk Contains Physically Stable Extracellular Vesicles Expressing Immunoregulatory TGF-β. PLoS One 10, e0121123 (2015*),* Bruno, MJ., Rusinova, R., Gleason, NJ. & Koeppe, R. E. Andersen OS Interactions of drugs and amphiphiles with membranes: modulation of lipid bilayer elastic properties by changes in acyl chain unsaturation and protonation. Faraday Discuss. 161, 461-480 (2013*)*]*.* A mean of 1410 proteins were identified from the proteome of every BP-EVs investigated (**Figure 5**). The number of total proteins identified from proteomes of BP-EVs ranged from 235 to 2822 proteins. These proteins were derived from the identification of an average of 15075 tryptic peptides generated during tryptic digestion of the proteomes of BP-EVs from every BP-EVs population investigated (**Figure 5**). The total number of tryptic peptides identified from BP-EVs ranged from 786 to 38719. This variability was associated to the complexity of the microbial population (Figure 5). Diversity and abundance of proteins was also investigated by bioinformatics and functional analysis. Relative quantifications of the abundance of membrane proteins identified in proteomes from BP-EVs revealed that enolases were the family of EVs membrane proteins more ubiquitously present across the analyzed BP-EVs and more abundant together with immunity-related membrane proteins and glycoproteins (Figure 6).

### Example 4

### BP-EVs closely resemble widely consumed food-derived EVs

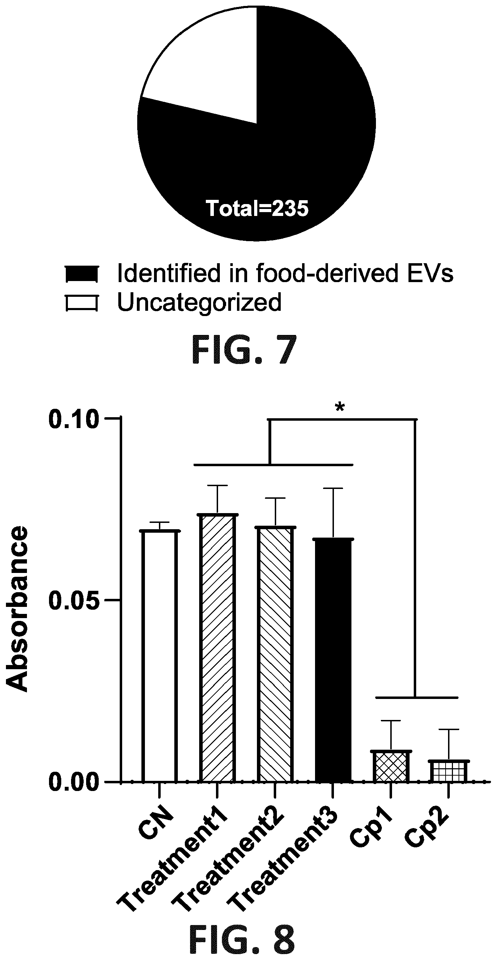

In order to comparatively analyze BP-EVs with their food-derived counterparts, we analyzed BP2-EVs proteome towards their respective previously reported bovine milk-derived EVs proteomes [Marco, M.L. et al. Health benefits of fermented foods: microbiota and beyond. Curr. Opin. Biotechnol. 44, 94-102 (2017*)]* (**Figure 6**). No available food proteomes were found for BP1 and BP3 sources and the available proteome associated with BP4 was too limited to establish robust comparative analyses. For BP2-EVs, 79% of the proteins consistently identified in our study were previously identified in bovine milk-derived EVs [Marco, M.L. et al. Health benefits of fermented foods: microbiota and beyond. Curr. Opin. Biotechnol. 44, 94-102 (2017*)],* as shown in **Figure 7**.

### Example 5

The potential ability to exert cytotoxicity of BP-EVs was assessed by performing the cell viability MTT assay in vitro using immortalized human colorectal Caco-2 cells. As shown in **Figure 8**, BP-EVs did not display significant decay on the absorbance obtained by the cytotoxicity MTT assay compared to negative (CN). The positive controls (CP) exerted a significant cytotoxicity, fact that give rise to a significant decrease of the absorbance measured in the MTT assay, indicating that BP-EVs did not exert a significant citotoxicity.

### Example 6

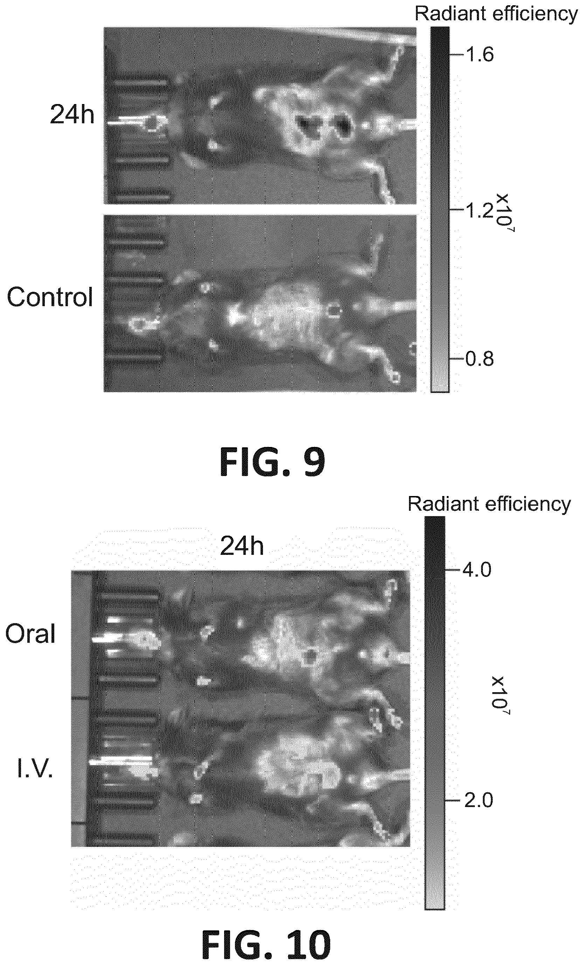

Biocompatible properties, including ability to target of specific organ tissues, circulation in biological fluids, ability to cross biological and cellular barriers and oral bioavailability, of BP-EVs were also investigated in vivo by whole-body fluorescent imaging followed by ex vivo fluorescent imaging validation. Overall abdominal biodistribution of fluorescent labelled BP-EVs was observed, and also fluorescence in the brain area, when analyzing the whole-body biodistribution of fluorescently labeled BP-EVs (**Figure 9**). Signal around the body was observed even 24 h post administration of fluorescently labeled BP-EVs. No significant differences were observed in the whole-body imaging study when comparing the oral and intravenous administration ways (**Fig**u**re 10**). Higher hepatic accumulation of BP-EVs was observed following to intravenous administration (i.v.) compared to oral administration.

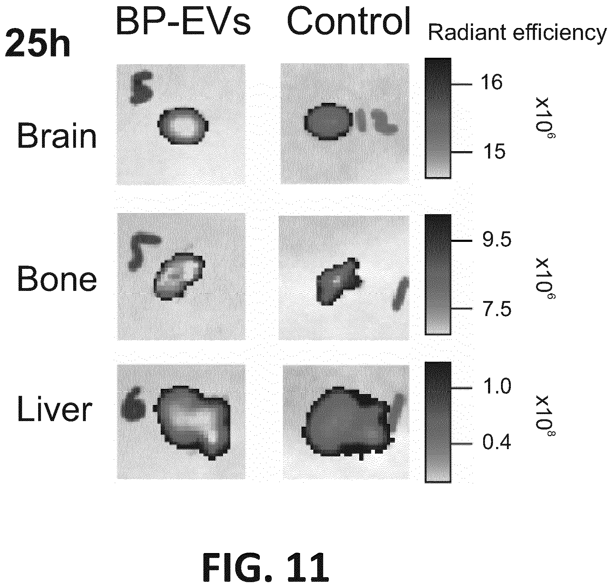

Ex vivo organ validation at 25h post-oral administration of BP-EVs, demonstrated the presence of fluorescently labeled BP-EVs in multiple organs, indicating differential targeting of these vesicles specifically in brain, liver and bone tissues (**Figure 11**). Although all analyzed BP-EVs sources demonstrated ability to specifically target brain tissues by ∼20% of the total biodistributed vesicles.

## Claims

1. Biocompatible extracellular vesicles, isolated from fermented food industry by-products, wherein the lipidome of the vesicles comprises at least eight families of lipids including fatty acyls (FA), glycerophospholipids (GP), glycerolipids (GL), sphingolipids (SP), saccharolipids (SL), polyketides (PK), sterol lipids (ST) and prenol lipids (PR), wherein the ratios between these lipids families are as follows:
| | **Lower** | **Upper** |
|---|---|---|
| | **range** | **range** |
| FA/GL | 0.4 | 1.4 |
| FA/GP | 0.2 | 0.4 |
| FA/PK | 1.3 | 3.4 |
| FA/PR | 4.2 | 7.1 |
| FA/SL | 61.2 | 124 |
| FA/SP | 0.9 | 3.0 |
| FA/ST | 0.9 | 1.6 |
and wherein the membrane proteome of said vesicles contains the metalloenzyme enolase and/or one or more of its isoforms.

2. Biocompatible extracellular vesicles, according to claim 1, wherein the fermented food industry by-products are generated during the production processes of fermented dairy products, fermented plant-based products and/or fermented beverages.

3. Biocompatible extracellular vesicles according to any of claims 1 or 2, wherein the diameter of the vesicles ranges from 30 to 950 nm.

4. Biocompatible extracellular vesicles according to claim 3, wherein the 50% of the vesicles have a diameter smaller than 200 nm.

5. Biocompatible extracellular vesicles according to any of claims 1 to 4 isolated by particle size filtration.

6. Biocompatible extracellular vesicles according to claim 5 wherein the particle size filtration technique is molecular weight cut off (MWCO).

7. Biocompatible extracellular vesicles according to any of claims 1 to 6 wherein said vesicles are intravenously and orally bioavailable.

8. Biocompatible extracellular vesicles according to any of claims 1 to 7 wherein the EVs are detected in brain more than 24 h after their oral or intravenous administration.

9. Composition comprising a population of biocompatible extracellular vesicles according to any of claims 1 to 8.

10. Use of fermented food industry by-products as source of biocompatible extracellular vesicles according to claims 1-8

11. Use of biocompatible extracellular vesicles, according to claims 1-8, as nanocarriers, nanovectors and/or nanocapsules for molecules delivery and/or encapsulation.

12. Method for the isolation of extracellular vesicles from fermented food industry by-products, according to any of claims 1 to 8, comprising the following steps:
a. Centrifugation, for liquid FFIBPs samples, at 4.000-12.000 x g, during 10-40 min, at 3-6°C, or homogenization followed by centrifugation, at 4.000-12.000 x g, during 10-40 min, at 3-6°C, for solid FFIBPs samples, to obtain pre-processed FFIBPs,
b. Centrifugation of pre-processed FFIBPs obtained in a) in a spin-filter of 200-500 kDa of pore size at 5.000-7.000 x g, for 15-45 min at 3-6°C,
c. Addition of PBS into the same spin-filter and centrifugation at 5.000-7.000 x g, for 15-45 min, at 3-6 °C,
d. Repeat step c), and
e. Collection of the concentrated BP-EVs sample remaining in the upper side of the filter in d).

13. Use of extracellular vesicles, according to any of claims 1 to 8, or a composition, according to claim 9, in the manufacture of a food and/or food supplement for animal or human use, a supplement for plants or a cosmetic product.

14. Composition according to claim 9 for use as a medicament.
